# EUROPEAN PATENT SPECIFICATION

(11) **EP 2 560 724 B1**
(45) Date of publication and mention of the grant of the patent: **04.09.2019**
(21) Application number: 11717417.7
(22) Date of filing: 18.04.2011
(51) Int. Cl.: A61M 27/00, A61M 1/00, G01C 15/00

(54) **ACCELEROMETER BASED LASER LEVELING DEVICE**
BESCHLEUNIGUNGSMESSERBASIERTES LASER-NIVELLIER-GERÄT
SYSTÈME DE NIVELLAGE PAR LASER FONDEE SUR UN ACCELEROMETRE

(30) Priority: 20.04.2010 US 763335
(43) Date of publication of application: 27.02.2013
(73) Proprietor: Medtronic Xomed, Inc., Jacksonville, FL 32216-0980 (US)
(72) Inventor: MURPHY, John M., Jacksonville, Florida 32259 (US)
(74) Representative: Pulieri, Gianluca Antonio
(86) International application number: PCT/US2011/032899
(87) International publication number: WO 2011/133469

(56) References cited:
- US-A- 5 280 789
- US-A- 5 772 625
- US-A1- 2007 028 470
- US-A1- 2007 293 847
- US-B1- 7 591 074

## Description

### Background

As well known in the medical arts, to relieve an undesirable accumulation of fluids from a part of the body, it is frequently necessary to provide a means for draining the fluid away from the body. Such is the case, for example, in the treatment of hydrocephalus, an ailment usually afflicting infants or children in which fluids which ought to drain away accumulate within the skull and thereby exert extreme pressure and skull deforming forces.

In treating hydrocephalus, cerebrospinal fluid accumulated in the brain ventricles is drained away by a catheter inserted into the ventricle through the skull, and the catheter is connected to a tube which conducts the fluid away from the brain either to another part of the body or to an external source. External drain systems typically are attached to intravenous (IV) poles and include a mounting assembly having a pole clamp and a scale, and a drip assembly adjustably fastened to the mounting assembly. The drip assembly typically includes a fluid-receiving graduated cylinder which often empties into a disposable bag.

A zero reference point on the skull is usually found using a laser leveling device. In particular, a zero point on the scale is aligned with this zero reference point on the skull. Additionally, a zero reference stop cock having fluid valves is also aligned with the zero reference point and usually attached to the pole clamp. In order to control the flow of cerebrospinal fluid and maintain the proper pressure in the brain ventricle, the drip assembly is elevated or lowered along the scale to encourage or reduce a gravity or pressure flow from the brain ventricles into the cylinder and/or bag.

Current laser leveling devices include a laser enclosed within a housing and one or more leveling tubes that indicate whether the housing (and thus the laser) is level with respect to gravity. The housing is attached to the scale so that the zero point on the scale, the zero point reference stop cock and the laser are all in fixed relation (typically aligned). A user inspects the leveling tube to determine whether the housing of the laser leveling device is level and/or whether rotation of the housing is needed to level the device. After the housing is level, the drainage system and/or patient can be positioned so that the laser is aligned with the zero reference point on the skull. Further examples of laser leveling devices are known from documents US 5,280,789 A, US 2007/0028470 A1 and US 7,591,074 B1.

### Summary of the disclosure

Aspects of concepts presented herein relate to a laser leveling device for use with an external medical drainage system. The laser leveling device includes a housing enclosing an accelerometer and a laser module. At least one level indicator is electrically coupled to the accelerometer to provide an indication of whether the housing is level with respect to gravity. In one particular aspect, the housing of the laser leveling device is rotatably coupled to the drainage system through a mounting bracket. A power source can further be enclosed within the housing to selectively provide power to the accelerometer, the laser module and the level indicator. Additionally, a switch can be electrically coupled to the power source such that, upon actuation of the switch, the power source provides power to the accelerometer and the laser module for a predetermined period of time.

In another aspect, a drainage system includes a catheter configured to be fluidly coupled to a patient and a drainage control component mounted to a panel spaced apart from the patient. The drainage control component is fluidly coupled to the catheter. A drip assembly is slidably mounted to the panel for selective positioning with respect to the drainage control component and fluidly coupled to the drainage control component. Furthermore, a laser leveling device is mounted to the panel and includes a housing, an accelerometer, a laser module and a level indicator. The level indicator is electrically coupled to the accelerometer to indicate a relative position of the housing with respect to gravity.

In an example not being part of the invention, a method for draining fluid from a patient includes fluidly coupling a catheter to the patient and fluidly connecting a drainage control component to the catheter. A laser leveling device is positioned in fixed relation to the drainage control component and includes a housing, an accelerometer, a laser module and a level indicator. The level indicator is electrically coupled to the accelerometer to indicate a relative position of the housing with respect to the gravity. The method also includes rotating the housing so as to be level with gravity and powering the laser module to form a laser beam. The laser beam is aligned with a reference point on the patient.

### Summary of the invention

In accordance with the present invention, there is provided a laser leveling system for use with a patient drainage system as defined in claim 1.

In addition, further advantageous embodiments follow from the dependent claims.

### Brief Description of the Drawings

FIG. 1 is a schematic view of an external drainage system.
FIG. 2 is a rear perspective view of a portion of the external drainage system of FIG. 1.
FIG. 3 is a front view of a laser leveling device.
FIG. 4 is a rear perspective view of the laser leveling device of FIG. 3.
FIG. 5 is a top view of the laser leveling device of FIG. 3.
FIG. 6 is a schematic diagram of components housed in the laser leveling device.

### Detailed Description

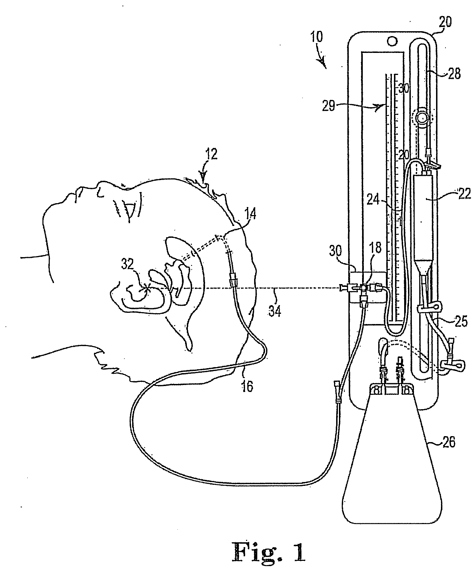

FIG. 1 is a schematic diagram of a drainage system 10 fluidly coupled to a patient 12. To fluidly couple the drainage system 10 to the patient 12, a catheter 14 is inserted into a skull of the patient to access a ventricle of the brain. Catheter 14 is fluidly connected to a first tubing segment 16 which leads to a drainage control component, herein embodied as a zero reference stop cock 18 mounted to a panel 20 of the drainage system 10. In particular, the zero reference stop cock 18 is mounted at a predetermined reference position on the panel 20. Zero reference stop cock 18 is also fluidly coupled to a drip assembly 22 through a second tubing segment 24.

Fluid from drip assembly 22 further passes through a third tubing segment 25 to a collection bag 26. Drip assembly 22 is positioned along panel 20 within a slot 28 such that drip assembly 22 is positioned at a level relative to zero reference stop cock 18 as prescribed by a physician. Indicia 29 on the panel provide a reference for determining the relative difference between zero reference stop cock 18 and drip assembly 22. As fluid pressure builds up within a head of patient 12, fluid is drained from the catheter 14 to the collection bag 26 based on the relative position of zero reference stop cock 18 and drip assembly 22.

To establish a desired fluid pressure within the head of patient 12, a laser leveling device 30 is used to align zero reference stop cock 18 with a zero reference point 32 marked on patient 12. In an example not being part of the invention, device 30 is aligned with zero reference stop cock 18 and zero reference point 32 is located at a top of an ear of the patient 12. The laser leveling device 30 emits a laser beam 34 (schematically shown) and a position (i.e., height) of drainage system 10 and/or patient 12 is adjusted such that beam 34 is aligned with zero reference point 32. As discussed below, laser leveling device includes at least one level indicator to insure that beam 34 is level with respect to gravity. In an example not being part of the invention, drainage system 10 is mounted to an IV pole (not shown) using a sliding mounting mechanism such that zero reference stop cock 18 and zero reference point 32 are positioned at the same height. One example mechanism for mounting drainage system 10 to a pole is shown and described in U.S. Patent No. 6,540,727.

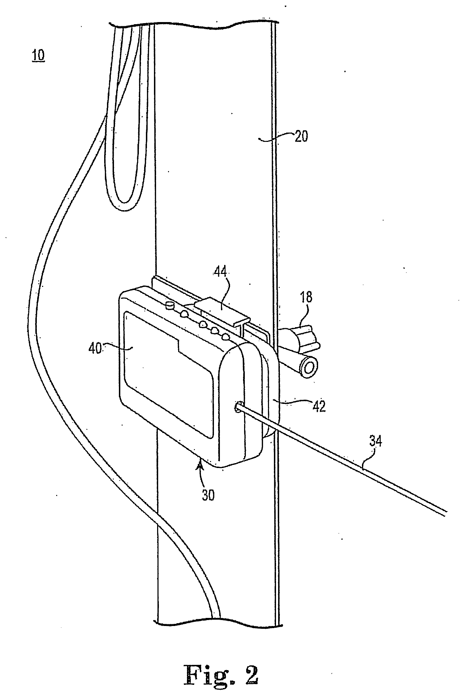

FIG. 2 is a rear view of a portion of drainage system 10 wherein laser leveling device 30 is rotatably mounted to panel 20. In particular, device 30 includes a housing 40 and a mounting bracket 42 coupled to the housing. Mounting bracket 42 includes a push button 44 for releasably securing mounting bracket 42 to the panel 20. Once mounting bracket 42 is secured to the panel 20, housing 40 can be rotated with respect to panel 20 and mounting bracket 42 so as to insure the housing 40 is level to gravity. Another example approach for mounting laser leveling device to panel 20 is shown and described in U.S. Patent No. 5,280,789. In any event, in an example not being part of the invention, housing 40 is configured to rotate about mounting bracket 42 a minimum of 180°, such that laser beam 34 can be oriented in parallel, opposite directions when level with gravity. For example, with reference to FIG. 1, if patient 12 were located on an opposite side of panel 20, laser beam 34 can be oriented in an opposite direction to that shown in FIG. 1 (e.g., to the right).

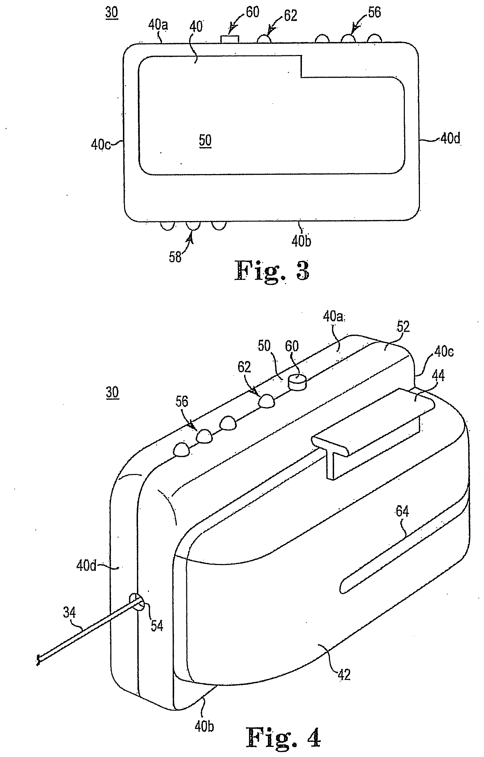

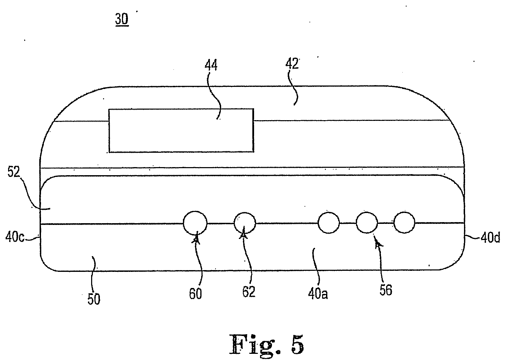

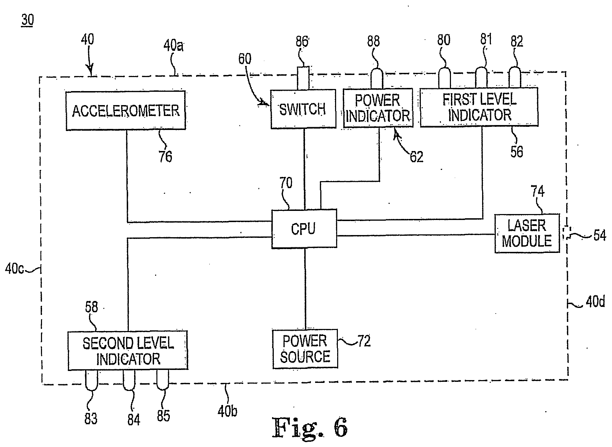

With reference to FIGS. 3-5, housing 40 is generally rectangular, defining a top 40a, bottom 40b and opposed sides 40c and 40d. Housing 40 further includes first and second housing portions 50 and 52 adapted to enclose electronic components of the laser leveling device 30, which are further described with respect to FIG. 6 below. Additionally, the housing 40 is configured to form openings for a laser aperture 54, a first level indicator 56, a second level indicator 58, a switch 60 and a power indicator 62. Laser aperture 54 is positioned on one side 40d of the housing 40 such that beam 34 is generally parallel to top 40a and bottom 40b. First level indicator 56 is positioned on the top 40a of the housing 40 whereas second level indicator 58 is positioned on the bottom 40b of the housing 40. As such, depending on a direction of orientation for laser beam 34, a level indicator will always be positioned in an upright fashion.

Each of the level indicators 56 and 58 are configured to provide a visual indication of whether housing 40 is level. In particular, the level indicator 56 and 58 provide an indication of whether top 40a, bottom 40b and laser beam 34 are parallel with respect to the ground. Switch 60 is provided to temporarily turn on the laser beam 34, wherein power indicator 62 provides an indication that power is provided to turn on the laser beam 34. In an example not being part of the invention, power indicator 62 can indicate a power level of a power source enclosed within housing 40 (e.g., battery level is low). Mounting bracket 42 further includes a groove 64 that cooperates with a tab on panel 20 of the drainage system 10 such that mounting bracket 42 is secured to panel 20.

Further reference is made to FIG. 6, which is a schematic block diagram of components at least partially enclosed within housing 40. In an example not being part of the invention, the components in FIG. 6 are electrically coupled together and mounted to a printed circuit board (not shown). As illustrated, first level indicator 56, second level indicator 58, switch 60 and power indicator 62 are partially exposed with respect to the housing 40 so as to provide visual and/or physical exterior exposure of the components. Enclosed within housing 40 are a CPU 70, a power source 72, a laser module 74 and an accelerometer 76. In an example not being part of the invention, CPU 70 is a MSP430™ microcontroller available from Texas Instruments of Dallas, Texas. Power source 72 can be a portable power source such as a battery and configured to provide power to each of the components in housing 40 and in particular, first level indicator 56, second level indicator 58, switch 60, power indicator 62, CPU 70, laser module 74 and accelerometer 76. Additionally, accelerometer 76 can be a three-axis accelerometer, for example part no. MMA7260QT from Freescale Semiconductor of Austin, Texas.

In the embodiment illustrated, first level indicator 56 includes three light emitting diodes (LEDs) 80, 81 and 82 that are exposed relative to the housing 40. Collectively, the LEDs provide an indication of whether the housing 40 is level with respect to gravity as a function of signals received from accelerometer 76. For example, outer LEDs 80 and 82 can be red LEDs whereas middle LED 81 is a green LED. If either LED 80 or LED 82 is lit, this is an indication that housing 40 is not level. If LED 81 is lit, this is an indication that housing 40 is level. In an example not being part of the invention, first level indicator 56 can include a single LED indicating housing 40 is level. Second level indicator 58 is similarly constructed to fist level indicator 56 and includes three LEDs 83, 84 and 85. Similar to LEDs 80-82, LEDs 83-85 can include two outer LEDS (e.g., LEDs 83 and 85) of a first color (e.g., red) and a middle LED (e.g., LED 84) of a second color (e.g., green) to indicate whether housing 40 is level. Switch 60 includes a push button 86 whereas power indicator 62 includes an LED 88 indicating whether the laser leveling device is operational.

To operate laser leveling device 30, push button 86 of switch 60 is actuated (i.e., depressed), which transmits a signal from switch 60 to CPU 70 indicating that the push button 86 has been depressed. CPU 70 then sends a signal to power indicator 62, laser module 74 and accelerometer 76 so as to turn on for a predetermined amount of time (e.g., approximately 20 seconds). During the predetermined amount of time, signals from accelerometer 76 are sent to CPU 70. CPU 70 interprets these signals and sends corresponding signals to first level indicator 56 and second level indicator 58. In particular, CPU 70 sends a signal to turn on one of the LEDs 80-82 of first level indicator 56 and one of the LEDs 83-85 of second level indicator 58.

For example, if accelerometer 76 indicates that housing 40 is tilted in a clockwise direction (as viewed in FIG. 6), CPU 70 will send signals to turn on LEDs 82 and 85. Conversely, if accelerometer 76 indicates that housing 40 is tilted in a counter-clockwise direction (as viewed in FIG. 6), CPU 70 will send signals to turn on LEDs 80 and 83. If accelerometer 76 indicates that housing 40 is level, CPU 70 will send a signal to turn on LEDs 81 and 84. Once a user determines that housing 40 is level as indicated by LEDs 81 and 84, laser beam 34 can be aligned with zero reference point 32 (FIG. 1). To ensure a fixed relationship between laser module 74 and accelerometer 76, a calibration process can be utilized.

## Claims

1. A laser leveling system for use with a patient drainage system (10), comprising:
a housing (40);
a mounting bracket (42) coupled to the housing (40) and configured to rotatably couple the housing (40) to the drainage system (10);
an accelerometer (76) enclosed within the housing (40);
a laser module (74) enclosed within the housing (40); and a first level indicator (56) positioned on a top of the housing (40) and electrically coupled to the accelerometer (76) to indicate a relative position of the housing (40) with respect to gravity,
wherein the first level indicator (56) includes first (80), second (81) and third (82) light emitting diodes, wherein the first (80) and third (82) of the light emitting diodes indicate that the housing (40) is not level and the second (81) of the light emitting diodes indicate that the housing (40) is level,
and in that the laser leveling system further comprises a second level indicator (58) positioned on a bottom (40b) of the housing (40) and electrically coupled to the accelerometer (76) to indicate a relative position of the housing (40) with respect to gravity, wherein the second level indicator (58) includes forth (83), fifth (84) and sixth (85) light emitting diodes, wherein the forth (83) and sixth (85) of the light emitting diodes indicate that the housing (40) is not level and the fifth (84) of the light emitting diodes indicate that the housing (40) is level,
and in that the first (80) and fourth (83) light emitting diodes are lit when the housing (40) is tilted in a first counter-clockwise direction with respect to gravity and
the third (82) and sixth (85) light emitting diodes are lit when the housing (40) is tilted in a second clockwise direction with respect to gravity opposite to the first direction.

2. The laser leveling system of claim 1, further comprising:
a power source (72) enclosed within the housing (40) to selectively provide power to the accelerometer (76), the laser module (74) and the first and second level indicators (56,58); and
a switch (60) electrically coupled to the power source (72) such that, upon actuation of the switch (60), the power source (72) provides power to the accelerometer (76) and the laser module (74) for a predetermined period of time.

3. The laser leveling system of claim 1 wherein the accelerometer (76) is a three-axis accelerometer.

4. The laser leveling system of claim 1, further comprising:
a catheter (14) configured to be fluidly coupled to a patient (12);
a drainage control component mounted to a panel (20) spaced apart from the patient (12), the drainage control component fluidly coupled to the catheter (14); and
a drip assembly (22) slidably mounted to the panel (20) for selective positioning with respect to the drainage control component and fluidly coupled to the drainage control component;
where the housing (40) of the laser leveling system is rotatably mounted to the panel (20).

5. The laser leveling system of claim 1,
wherein the second (81) light emitting diode is a middle light emitting diode between outer first (80) and third (82) light emitting diodes; and
wherein the fifth (84) light emitting diode is a middle light emitting diode between outer fourth (83) and sixth (85) light emitting diodes.

6. The laser leveling system of claim 5,
wherein the second (81) and fifth (84) light emitting diodes are green light emitting diodes; and
the first (80), third (82), fourth (83) and sixth (85) light emitting diodes are red light emitting diodes.

## Patentansprüche

1. Laser-Nivelliersystem für die Verwendung mit einem Drainagesystem für Patienten (10), umfassend:
ein Gehäuse (40);
eine Montagehalterung (42), die mit dem Gehäuse (40) gekoppelt ist und dazu konfiguriert ist, das Gehäuse (40) mit dem Drainagesystem (10) drehbar zu koppeln;
einen Beschleunigungsmesser (76), der in dem Gehäuse (40) eingeschlossen ist;
ein Lasermodul (74), das in dem Gehäuse (40) eingeschlossen ist; und
eine erste Füllstandsanzeige (56), die an einer Oberseite des Gehäuses (40) positioniert und elektrisch mit dem Beschleunigungsmesser (76) gekoppelt ist, um eine relative Position des Gehäuses (40) in Bezug auf die Schwerkraft anzuzeigen, wobei die erste Füllstandsanzeige (56) eine erste (80), eine zweite (81) und eine dritte (82) Leuchtdiode umfasst, wobei die erste (80) und die dritte (82) Leuchtdiode anzeigen, dass das Gehäuse (40) nicht nivelliert ist, und die zweite Leuchtdiode (81) anzeigt, dass das Gehäuse (40) nivelliert ist, **dadurch gekennzeichnet, dass** das Laser-Nivelliersystem ferner eine zweite Nivellieranzeige (58) umfasst, die an einem Boden (40b) des Gehäuses (40) gelagert und elektrisch mit dem Beschleunigungsmesser gekoppelt ist (76), um eine relative Position des Gehäuses (40) in Bezug auf die Schwerkraft anzuzeigen, wobei die zweite Füllstandsanzeige (58) eine vierte (83), eine fünfte (84) und eine sechste (85) Leuchtdiode aufweist, wobei die vierte (83) und die sechste (85) Leuchtdiode anzeigen, dass das Gehäuse (40) nicht nivelliert ist, und die fünfte (84) Leuchtdiode anzeigt, dass das Gehäuse (40) nivelliert ist, und wobei die erste (80) und die vierte (83) Leuchtdiode leuchten, wenn das Gehäuse (40) in einer ersten dem Uhrzeigersinn entgegengesetzten Richtung in Bezug auf die Schwerkraft geneigt ist, und die dritte (82) und die sechste (85) Leuchtdiode leuchten, wenn das Gehäuse (40) in Bezug auf die Schwerkraft entgegen der ersten Richtung in eine zweite Richtung im Uhrzeigersinn geneigt ist.

2. Laser-Nivelliersystem nach Anspruch 1, ferner Folgendes umfassend:
eine Stromquelle (72), die in dem Gehäuse (40) eingeschlossen ist, um selektiv den Beschleunigungsmesser (76), das Lasermodul (74) und die erste und zweite Füllstandsanzeige (56, 58) mit Strom zu versorgen; und
einen Schalter (60), der elektrisch mit der Stromquelle (72) derart verbunden ist, dass bei Betätigen des Schalters (60) die Stromquelle (72) den Beschleunigungsmesser (76) und das Lasermodul (74) für eine vorbestimmte Zeitspanne mit Strom versorgt.

3. Laser-Nivelliersystem nach Anspruch 1, wobei der Beschleunigungsmesser (76) ein Drei-Achsen-Beschleunigungsmesser ist.

4. Laser-Nivelliersystem nach Anspruch 1, ferner Folgendes umfassend:
einen Katheter (14), der konfiguriert ist, um mit einem Patienten (12) fluidisch verbunden zu werden;
eine Drainagekontrollkomponente, die an eine Platte (20) angebracht ist, die von dem Patienten (12) beabstandet ist, wobei die Drainagekontrollkomponente mit dem Katheter (14) fluidisch gekoppelt ist; und
eine Tropfanordnung (22), die zur selektiven Positionierung in Bezug auf die Drainagesteuerkomponente verschiebbar an der Platte (20) angebracht ist und mit der Drainagekontrollkomponente fluidisch gekoppelt ist;
wobei das Gehäuse (40) des Laser-Nivelliersystems drehbar an der Platte (20) angebracht ist.

5. Laser-Nivelliersystem nach Anspruch 1, wobei die zweite (81) Leuchtdiode eine mittlere Leuchtdiode zwischen der äußeren ersten (80) und dritten (82) Leuchtdiode ist; und
wobei die fünfte (84) Leuchtdiode eine mittlere Leuchtdiode zwischen der äußeren vierten (83) und der sechsten (85) Leuchtdiode ist.

6. Laser-Nivelliersystem nach Anspruch 5, wobei die zweite (81) und fünfte (84) Leuchtdiode grüne Leuchtdioden sind; und
die erste (80), die dritte (82), die vierte (83) und die sechste (85) Leuchtdiode rote Leuchtdioden sind.

## Revendications

1. Système de nivellage par laser, destiné à être utilisé avec un système de drainage de patient (10), comprenant :
un boîtier (40) ;
un support de montage (42) couplé au boîtier (40) et configuré pour coupler de manière rotative le boîtier (40) au système de drainage (10) ;
un accéléromètre (76) enfermé à l'intérieur du boîtier (40) ;
un module laser (74) enfermé à l'intérieur du boîtier (40) ; et
un premier indicateur de niveau (56) placé sur une partie supérieure du boîtier (40) et couplé électriquement à l'accéléromètre (76) pour indiquer une position relative du boîtier (40) par rapport à la gravité, le premier indicateur de niveau (56) comprenant des première (80), deuxième (81) et troisième (82) diodes électroluminescentes, les première (80) et troisième (82) diodes électroluminescentes indiquant que le boîtier (40) n'est pas uniforme et la deuxième (81) diode électroluminescente indiquant que le boîtier (40) est uniforme, le système de nivellage par laser comprenant en outre un second indicateur de niveau (58) placé sur une partie inférieure (40b) du boîtier (40) et couplé électriquement à l'accéléromètre (76) pour indiquer une position relative du boîtier (40) par rapport à la gravité, le second indicateur de niveau (58) comprenant des quatrième (83), cinquième (84) et sixième (85) diodes électroluminescentes, les quatrième (83) et sixième (85) diodes électroluminescentes indiquant que le boîtier (40) n'est pas uniforme et la cinquième (84) diode électroluminescente indiquant que le boîtier (40) est uniforme, les première (80) et quatrième (83) diodes électroluminescentes s'allumant lorsque le boîtier (40) est incliné dans un premier sens anti-horaire par rapport à la gravité, et les troisième (82) et sixième (85) diodes électroluminescentes s'allumant lorsque le boîtier (40) est incliné dans un second sens horaire opposé au premier sens, par rapport à la gravité.

2. Système de nivellage par laser selon la revendication 1, comprenant en outre :
une source d'alimentation (72) enfermée à l'intérieur du boîtier (40), destinée à alimenter de manière sélective l'accéléromètre (76), le module laser (74) et les premier et second indicateurs de niveau (56, 58) ; et
un commutateur (60) couplé électriquement à la source d'alimentation (72) de sorte que, lors de l'actionnement du commutateur (60), la source d'alimentation (72) alimente l'accéléromètre (76) et le module laser (74) pendant une durée prédéterminée.

3. Système de nivellage par laser selon la revendication 1, dans lequel l'accéléromètre (76) est un accéléromètre à trois axes.

4. Système de nivellage par laser selon la revendication 1, comprenant en outre :
un cathéter (14) configuré pour en communication fluidique avec un patient (12) ;
un composant de commande de drainage monté sur un panneau (20) à distance du patient (12), le composant de commande de drainage étant couplé de manière fluide au cathéter (14) ; et
un ensemble d'égouttement (22) monté de manière coulissante sur le panneau (20) pour un positionnement sélectif par rapport au composant de commande de drainage, et couplé de manière fluidique au composant de commande de drainage ;
le boîtier (40) du système de nivellage par laser étant monté de manière rotative sur le panneau (20).

5. Système de nivellage par laser selon la revendication 1, dans lequel la deuxième (81) diode électroluminescente est une diode électroluminescente centrale située entre les première (80) et troisième (82) diodes électroluminescentes extérieures ; et
la cinquième (84) diode électroluminescente est une diode électroluminescente centrale située entre les quatrième (83) et sixième (85) diodes électroluminescentes extérieures.

6. Système de nivellage par laser selon la revendication 5, dans lequel les deuxième (81) et cinquième (84) diodes électroluminescentes sont des diodes électroluminescentes vertes ; et
les première (80), troisième (82), quatrième (83) et sixième (85) diodes électroluminescentes sont des diodes électroluminescentes rouges.
